# EUROPEAN PATENT SPECIFICATION

(11) **EP 1 912 616 B1**
(45) Date of publication and mention of the grant of the patent: **11.05.2016**
(21) Application number: 06792600.6
(22) Date of filing: 28.07.2006
(51) Int. Cl.: A61K 8/97, A61K 8/35, A61Q 19/10

(54) **OTOLOGICAL SOLUTIONS CONTAINING LAPACHO EXTRACT**
LAPACHO-EXTRAKT ENTHALTENDE OHRENREINIGUNGSLÖSUNGEN
SOLUTIONS OTOLOGIQUES CONTENANT DES EXTRAITS DE PAU D'ARCO

(30) Priority: 29.07.2005 IT TO20050533
(43) Date of publication of application: 23.04.2008
(73) Proprietor: Euro-Pharma S.r.l., 10143 Torino (IT)
(72) Inventor: MISCIOSCIA, Mario, I-10126 Torino (IT)
(74) Representative: Gervasi, Gemma
(86) International application number: PCT/EP2006/064780
(87) International publication number: WO 2007/014912

(56) References cited:
- EP-B1- 1 124 816
- WO-A-95/19108
- WO-A-2004/035071

## Description

### Field of the invention

The present invention refers to products for the body hygiene, and in particular to otological solutions, comprising basically Lapacho extracts in polar or non-polar solvents.

The invention refers also to the preparation of such products and their use for body hygiene.

### State of the art

***Tabebuia impetiginosa* (a.k.a T. *avellanedae*)** is tree species in the family Bignoniaceae (catalpa family) native to rainforests throughout Central and South America.

Mainly exported from Argentina, Paraguay, and Brazil, it is commonly known as: lapacho, pau d'arco, taheebo, and ipe roxo.

Since more than one thousand years the plant is used for medical purposes by indigenous populations which utilize in particular the internal part of the bark obtaining extracts useful in the treatment of various diseases.

Many studies have been performed in order to identify the pharmacological potential of the Lapacho extracts and various active principle have been isolated. Essentially 18 different quinones have been identified (see Edward H. Oswald - British Journal of Phytotherapy, Vol. 3, n. 3 (1993/1994*).*

The pharmacological properties of various extracts have been studied and it was proved that they posses antibiotic, antiviral, antimicotic, and antiseptic activity which made them appropriated i.e. in the treatment of mouth, nose and throat infections.

### Detailed description of the invention

It was now surprisingly found that the traditional extracts of Lapacho bark, either obtained with polar or non polar solvents, yield the particular property to disaggregate, solubilise and remove earwax and, therefore, they can be used in otological solutions useful for the cleansing of the auricular duct.

According to the present invention, these products can be easily prepared through hot-extraction with polar solvents (such as water, alcohol, acetone) or non polar solvents (such as ether, etc.) or their mixtures, of the Lapacho bark and then by filtering the suspension (following well-known methods).

When preferred, quinones contained in the Lapacho bark (which are not water soluble, i.e., quercetin) could be added to the water extract obtained from the bark, according to the method described in the Italian patent no. 1.304.304 of the same applicant.

According to this latter patent, water-insoluble quinones can be added to the aqueous solution either as commercially available products or recovering them from the bark after the extraction with hot-water, for example by salifying them in order to make them soluble in water as widely described in the above said patent.

The latter instance obviously presents the advantage that all naturally present quinones contained in the Lapacho bark are available, even those not commercially available.

The obtained product is then processed following the known techniques to obtain the final mixture ready to be used.

Usually, the extract is concentrated up to about 10%; however, different concentrations could be used.

Besides Lapacho extract, the otological formulations described in the present invention can also contain other active ingredients, such as anti-inflammatory and/or antiseptic ingredients together with possible preservatives (i.e., nipagine).

Cosmetic excipients known in the otological field (in particular those used for the cleansing of the auricular duct) can be added to the above solution, such as glycerine, urea peroxide, PEG, sodium lauret sarcosinate, citric acid, dibasic sodium phosphate, polysorbytan monostearate, hydroxypropylmethyl cellulose, etc.

When preferred, the solution containing Lapacho extract can be added with other vegetal extracts, such as camomile, almond oil, other herbal extracts with anti-inflammatory activity, Flavonoids, vitamins, etc.

Otological formulations prepared according to the present invention can comprise, for example:

| | |
|---|---|
| Lapacho extract | 5% - 90%, |
| water | 5% - 90%, |
| herbal extracts | 5% - 90%. |

A formulation according to the present invention may comprise, for example:

| | |
|---|---|
| Lapacho extract | 10% |
| water | 89.8% |
| Nipagine | 0.2% |
| Sodium hydroxyde q.b. a pH 6.5 | |

The present invention will then better described with the following examples.

### Example 1

10 kg micronised Lapacho bark (Tabebuia species) are warmed at 65-70 °C for 1-3 hours in 90 kg aqueous solvent (controlled pH value = 7.5) under continuous air-free stirring. Thereafter the solution is cooled at 20°C, then filtered. Filtrate is collected and preservatives (0.2% paraben mixture) are added. The above solution can be utilised to prepare final formulations.

### Example 2

10 kg micronised Lapacho bark (Tabebuia species) are warmed at 65-70°C for 1-3 hours in 90 kg aqueous solvent (controlled pH value = 7.5) under continuous air-free stirring. Thereafter the solution is cooled at 20°C, then filtered. Filtrate is collected and preservatives (0.2% paraben mixture) are added. The above solution is then added with ethanolic and/or glycerolic and/or lyophilic extracts at a concentration of 1 - 70% (p/p). The above solution can be utilised to prepare final formulations.

As reported previously, Lapacho extract can be prepared by means of solvents different from water (either polar or non polar) or by means of appropriate solvent mixtures (either polar or non polar), in analogy to what has been described above and using methods already described in the scientific literature.

The solution obtained according the above procedures can be formulated in the form of drops, bottles and/or strips containing variable amounts of liquid, different sprays with or without gaseous propellant, to compliance any preferred method of use.

## Claims

1. Otological formulations for use in the treatment for cleaning the auricular duct by dissolving, disaggregating and removing ear wax **characterised in that** said otological formulations comprise Lapacho extract.

2. Otological formulations according to Claim 1 where said extracts are obtained by treating Lapacho bark with water.

3. Otological formulations according to Claim1 including:
| | |
|---|---|
| Lapacho extract | 5% - 90%, |
| water | 5% - 90%, |
| vegetable extracts | 5% - 90%. |

4. Otological formulations according to Claim 1 including:
| | |
|---|---|
| Lapacho extract | 10.0% |
| water | 89.8% |
| nipagine | 0.2% |
| NaOH up to pH 6.5 | |

5. Otological formulations according to Claim 1 in the form of drops, bottles and/or strips containing variable amounts of liquid, different sprays with or without gaseous propellant, rubber-syringes either disposable or not.

## Patentansprüche

1. Otologische Rezepturen, zur Anwendung in der Behandlung zur Reinigung des Aurikularkanals durch Auflösen, Zerlegen und Entfernen von Ohrenschmalz, **dadurch gekennzeichnet, dass** die genannte otologische Rezeptur Lapachoextrakt enthält.

2. Otologische Rezepturen nach Anspruch 1, in welchen die Extrakte durch Behandlung von Lapachorinde mit Wasser gewonnen werden.

3. Otologische Rezepturen nach Anspruch 1, umfassend
| |
|---|
| Lapachoextrakt 5% - 90%, |
| Wasser 5% - 90%, |
| pflanzliche Extrakte 5% - 90%. |

4. Otologische Rezepturen nach Anspruch 1, umfassend
| |
|---|
| Lapachoextrakt 10.0% |
| Wasser 89.8% |
| Nipagine 0.2% |
| NaOH bis zu einem pH von 6.5. |

5. Otologische Rezepturen nach Anspruch 1 in Form von Drops, Flaschen und/oder Streifen, welche variable Flüssigkeitsmengen enthalten, unterschiedlichen Sprays mit oder ohne gasförmiges Treibmittel, Gummispritzen, entweder wegwerfbar oder nicht.

## Revendications

1. Solutions otologiques pour une utilisation dans le traitement pour nettoyer le conduit auriculaire en dissolvant, désagrégeant et enlevant du cérumen, **caractérisées en ce que** lesdites solutions otologiques comprennent des extraits de pau d'arco (Lapacho).

2. Solutions otologiques selon la revendication 1, **caractérisées en ce que** les extraits de pau d'arco sont obtenus en traitant des écorces de pau d'arco avec de l'eau.

3. Solutions otologiques selon la revendication 1, **caractérisées en ce qu'**elles comprennent :
| | |
|---|---|
| extraits de pau d'arco | 5% - 90%, |
| eau | 5% - 90%, |
| extraits de légumes | 5% - 90%. |

4. Solutions otologiques selon la revendication 1, **caractérisées en ce qu'**elles comprennent :
| | |
|---|---|
| extraits de pau d'arco | 10,0%, |
| eau | 89,8%, |
| nipagine | 0,2% |
| NaOH jusqu'à pH 6,5 | |

5. Solutions otologiques selon la revendication 1 sous la forme de dragées, bouteilles et/ou lamelles contenant différentes quantités de liquide, différents sprays avec ou sans propulsant gazeux, seringues en caoutchouc jetables ou non.
